# EUROPÄISCHE PATENTSCHRIFT

(11) **EP 0 695 193 B1**
(45) Veröffentlichungstag und Bekanntmachung des Hinweises auf die Patenterteilung: **10.02.1999**
(21) Anmeldenummer: 94915529.5
(22) Anmeldetag: 22.04.1994
(51) Int. Cl.: A61K 47/22, A61K 47/24

(54) **TRANSDERMALE WIRKSTOFFZUBEREITUNG**
TRANSDERMAL ACTIVE-SUBSTANCE PREPARATION
PREPARATION TRANSDERMIQUE DE PRINCIPES ACTIFS

(30) Priorität: 23.04.1993 DE 4313402
(43) Veröffentlichungstag der Anmeldung: 07.02.1996
(73) Patentinhaber: HEXAL AG, D-83607 Holzkirchen (DE)
(72) Erfinder: FISCHER, Wilfried, D-83607 Holzkirchen (DE); STRÜNGMANN, Thomas, D-83607 Holzkirchen (DE)
(74) Vertreter: Boeters, Hans Dietrich, Dr.
(86) Internationale Anmeldenummer: EP9401259
(87) Internationale Veröffentlichungsnummer: WO9425069

(56) Entgegenhaltungen:
- EP-A- 0 343 694
- EP-A- 0 372 527
- EP-A- 0 383 162
- WO-A-90/06736
- JOURNAL OF CONTROLLED RELEASE, Bd.14, Nr.3, Dezember 1990, AMSTERDAM (NL) Seiten 243 - 252, XP165642 M. MAHJOUR ET AL. 'effect of egg yolk lecithins and commercial soybean lecithins on in vitro skin permeation of drugs'
- DIE PHARMAZIE, Bd.47, Nr.5, Mai 1992, BERLIN (DE) Seiten 386 - 387, XP287884 J. SZULC ET AL. 'herstellung von liposomen mit den vitaminen a und e'

## Beschreibung

Die transdermale Verabreichung von Wirkstoffen, beispielsweise entzündungshemmenden oder antithrombotischen Wirkstoffen, ist in Fällen angebracht, in denen eine hohe örtliche Konzentration der Wirkstoffe im Gewebe unter der intakten Haut erforderlich ist, während die orale oder parenterale systemische Verabreichung zu unerwünschten systemischen Nebeneffekten führt oder diese Verabreichungsart nicht die erforderliche örtliche Konzentration bieten kann. Wirkstoffe, wie Heparin-natrium werden im allgemeinen wegen geringer Hautpermeabilität in hohen Konzentrationen topisch eingesetzt. Die intestinale Absorption dieses Wirkstoffs ist vernachlässigbar. Systemische Effekte sind bei peripheren Verletzungen unerwünscht. Viele topische Wirkstofformulierungen können die Haut dort, wo sie aufgebracht werden, sensibilisieren, austrocknen oder sogar schädigen. Wasserhaltige Cremes oder Salben, die oft dazu vorgesehen sind, einige dieser unerwünschten Nebeneffekte zu vermeiden, müssen mikrobiologische Konservierungsmittel enthalten, die ihrerseits oft zu einer Hautsensibilisierung führen. Es besteht daher ein Bedürfnis für sichere, nicht-irritierende und zusätzlich die Haut schützende Absorptionsbeschleuniger, die für Wirkstoffe mit geringem Hautpermeationsvermögen vorteilhaft sind, um die Wirkstoffkonzentration im Zielgewebe zu erhöhen. Diclofenac-natrium ist ein Beispiel für einen außerordentlich oft eingesetzten nicht-steroidalen entzündungshemmenden Wirkstoff (NSAID), und zwar im Hinblick auf seine problemlose und gut-etablierte klinische Wirksamkeit. Um die bekannten örtlichen mucosalen Irritationen nach oraler Einnahme zu mindern, sind topische Verabreichungsformen bei regionalen rheumatischen Beschwerden für einen chronischen Einsatz erwünscht. Ferner kann der Metabolismus bei der ersten Passage nach peroraler Verabreichung, der in der Leber auftritt und zu einer partiellen Inaktivierung führt, durch topische Verabreichung vermieden werden. Für die folgenden Ausführungen wurde Diclofenac als Beispiel für Wirkstoffe mit geringem Hautpermeationsvermögen gewählt.

Die Wirksamkeit von topischen Diclofenac-natrium-Zubereitungen hängt stark von der Fähigkeit der Zubereitung ab, den Wirkstoff die intakte Haut durchdringen zu lassen. Insbesondere stellt die Hornhaut eine Barriere für jede Wirkstoffpermeation der Haut und das Eindringen in das darunterliegende entzündete Gewebe dar. Da die Permeabilität intakter Haut für Diclofenac-natrium gering ist, sind verschiedene Versuche unternommen worden, die Hautpermeation durch Einsatz verschiedener Salze zu erhöhen. IL-A-62 160 beschreibt Aminsalze von Diclofenac für eine erhöhte Hautpenetration. DE-A-3 336 047 (= GB-A-2 128 087) beschreibt eine topische Zubereitung auf Basis einer Öl-in-Wasser Emulsion, die das Diethylammoniumsalz von Diclofenac enthält. Dieses Salz ist lipophiler als das Natriumsalz und kann die Haut mit einer höheren Rate permeieren. Der Einsatz dieses Salzes ist jedoch nicht risikolos. Es ist bekannt, daß sekundäre Amine hochkanzerogene Nitrosamine bilden. Die Zubereitung enthält zusätzlich Diethylamin als Neutralisierungsmittel für Acrylsäure, die als Gelbildungsmittel für die Gelzubereitung wirkt. Da sekundäre Amine diese potentiellen Nebenwirkungen besitzen, haben die für eine Registrierung zuständigen deutschen Behörden (BGA) empfohlen, diese Amine bei kosmetischen und pharmazeutischen Zubereitungen zu ersetzen. EP-A-0 372 527 beschreibt die Verwendung eines Hydroxyethylpyrrolidinsalzes von Diclofenac mit einer höheren Wasserlöslichkeit als beim Natriumsalz. Dieses Salz wurde mit verschiedenen Absorptionsförderern kombiniert, beispielsweise ethoxylierten Glyceriden, Lanolinestern oder Lecithin. Nur in Verbindung mit diesen neuen Salzen waren die Absorptionspromotoren in der Lage, die Absorption in einem derartigen Ausmaß wie das Diethylaminsalz zu beschleunigen. Man vergleiche dazu Clin. Tri. J., 26 (1989) 304 - 309 (Galzigna et al.: percutaneous adsorption of diclofenac after topical application, Two different gel formulations). JP-A-02.049 722 beschreibt die Verwendung von Ammoniak als Neutralisierungsmittel für Polyacrylsäuregele mit einem Gehalt an Diclofenac-natrium. Wenn man als Neutralisierungsmittel nicht Ammoniak nahm, sondern ein Alkylamin, beispielsweise Monoethylamin, fiel die Hautpermeation *in vitro* von 44,8 auf 1,7 %. EP-A-0 498 011 beschreibt Rubidium- oder Cäsiumsalze von Diclofenac mit einer höheren Hautpermeation *in vitro* als beim Natriumsalz. Die Verwendung dieser Salze ist jedoch kritisch, da beide Ionen ihre eigenen pharmakologischen Wirkungen entfalten. So treten antidepressive Effekte auf, und insbesondere erhöht Cäsium die zerebralen Konzentrationen von Serotonin und Tryptophan. Seine sehr lange Eliminierungs-Halbzeit (50 bis 100 Tage) kann zu einer Anhäufung im Körper nach chronischer Verabreichung führen. Rubidium zeigt gleichfalls eine lange Eliminierungshalbzeit (40 Tage) und führt zu einer Langzeitakkumulation. In einigen Fällen kann es die zentral nervöse Aktivität und die Aggressivität erhöhen. EP-A-0 245 126 beschreibt das Calciumsalz von Diclofenac in Kombination mit toxikologisch riskanten Absorptionspromotoren, wie DMSO und Azone. Neben dem Einsatz neuer Salze von Diclofenac sind verschiedene Zubereitungen mit einem Gehalt an synthetischen Absorptionspromotoren entwickelt worden. So beschreibt JP-A-01.013 020 Diclofenac-natrium-Emulsionen für den topischen Einsatz, die Dialkylcarboxylate und Fettsäuren enthalten. Zusätzlich ist ein Schutzmittel zuzugeben, um die mikrobiologische Kontamination herabzusetzen. Nach WO-A-88/04 938 werden heterozyklische Penetrationsbeschleuniger, wie Imidazoline oder Oxazoline, für topische Pharmazeutika von Diclofenac eingesetzt. Nachteile derartiger Beschleuniger sind jedoch ihre mögliche Hautirritation und Sensibilisierungen. US-A-4 670 254 beschreibt Diclofenac-natrium-Gele, die eine hohe Menge an Ethanol und Propylenglycol enthalten. Ethanol ist ein bekannter Absorptionspromotor für verschiedene Wirkstoffe. Ethanol und Propylenglykol sind Lösungsmittel für Lipide und zeigen den Nachteil, daß sie die Haut austrocknen. JP-A-60.146 823 verwendet Nikotinate als Absorptionsbeschleuniger. Als Wirkstoffe führen sie zu erhöhter Zirkulation und erhöhtem Blutfluß und beschleunigen so den Transport von der Applikationsstelle aus. Sie führen zu Wärmesensationen und störenden Nebeneffekten. EP-A-0 147 476 verwendet Glykole und Salizylate oder Pfefferminzöl oder Menthol als Absorptionsbeschleuniger. Diese Substanzen können jedoch Hautirritationen bewirken, und Pfefferminzöl oder Menthol können zu einem Brennen oder zur Sensibilisierung der Haut führen.

Ferner ist bekannt, daß Phospholipide den Feuchtigkeitsgehalt der Haut und die Permeation von Wirkstoffen durch die Haut erhöhen können. Die Literaturaussagen sind für Diclofenac-natrium allerdings widersprüchlich. Nach Chem. Pharm. Bull., 35 (1987) 3807 - 3812 (Nishihata et al.) erhöht sich die Permeation *in vitro* durch Rattenhaut, wenn Diclofenac in einem wässerigen Gel mit einem Gehalt an hydrierten Sojaphospholipiden eingesetzt wird (30 % Phosphatidylcholin und 70 % Phosphatidylethanolamin; Jodwert circa 6 %). Die nach 7 h permeierte Menge war beim Gel mit 0,5 % hydrierten Phospholipiden 5-mal höher als in Lösung ohne Phospholipide. Die Wirkstoffkonzentation betrug 1,25 % bei jedem Versuch. Andererseits sollen nach 2nd Liposome Research Days, NL-Leiden, June 1992, page 5 (Enghausen & Müller-Goymann) Phospholipide *in vitro* keine beschleunigende Wirkung zeigen, selbst wenn man eine isolierte Stratumhornhaut mit Phospholipiden vorbehandelte. Dieses zuletzt angeführte Ergebnis stimmt mit Beobachtungen der Erfinder überein (Vergleichsbeispiel 1).

Schließlich ist aus EP-A-0 343 694 eine Wirkstoffzubereitung mit einem Gehalt an Phospholipid bekannt, die Wasser, D-α-Tocopherolkonzentrat und Heparin-Na enthält. Mit dieser bekannten Lehre soll eine Wirkstoffzubereitung vorgesehen werden, bei der die membranabdichtende Wirkung von Vitamin E ausgenutzt wird.

Der Erfindung liegt die Aufgabe zugrunde, für transdermale Wirkstoffzubereitungen mit einem Gehalt an Phospholipiden das Permeationsvermögen zu verbessern.

Erfindungsgemäß wurde überraschenderweise festgestellt, daß sich bei derartigen transdermalen Wirkstoffzubereitungen die Permeationsfähigkeit durch die Zugabe von Vitamin E (α-Tocopherol) oder durch Zugabe von Vitamin-E-Derivaten vorteilhaft verbessern läßt.

Die der Erfindung zugrundeliegende Aufgabe wird durch eine Wirkstoffzubereitung mit einem Gehalt an Phospolipid gelöst, wobei diese Zubereitung dadurch gekennzeichnet ist, daß es sich um eine transdermale Wirkstoffzubereitung handelt, und zwar mit einem Gehalt an
- Wasser
- einem aliphatischen C₁₋₆-Alkohol im Bereich von 1 bis 50 % (bezogen auf das Gesamtgewicht der Zubereitung), wobei 7,2 % und weniger ausgenommen sind,
- α-Tocopherol oder α-Tocopherol-Derivat und
- einem Hormon, Diclofenac-Na oder Heparin-Na als Wirkstoff.

Die erfindungsgemäße Wirkstoffzubereitung kann durch einen Gehalt an α-Tocopherol oder α-Tocopherol-Derivat im Bereich von 0,1 bis 10 % und insbesondere 0,5 bis 7 % gekennzeichnet sein, bezogen auf das Gesamtgewicht der Zubereitung.

Ferner kann die erfindungsgemäße Wirkstoffzubereitung durch einen aliphatischen C₂₋₄-Alkohol als aliphatischen C₁₋₆-Alkohol gekennzeichnet sein, gegebenenfalls neben einem weiteren üblichen Alkohol.

Ferner kann die erfindungsgemäße Wirkstoffzubereitung durch einen Gehalt an Phospholipid im Bereich von 0,5 bis 20 % gekennzeichnet sein, bezogen auf das Gesamtgewicht der Zubereitung.

Ferner kann die erfindungsgemäße Zubereitung durch Liposome auf Basis des Phospholipids gekennzeichnet sein.

Bei den Wirkstoffzubereitungen kann es sich um topische Lösungen, Schäume, Sprays, Gele, Pasten, Salben, Emulsionen und liposomale Dispersionen handeln. Da bei niedrigen Alkoholkonzentrationen eine antimikrobische Aktivität gegeben ist, ist der Einsatz zusätzlicher Konservierungsmittel nicht erforderlich. Da erfindungsgemäß Tocopherol oder ein Tocopherol-Derivat vorgesehen wird, sind weitere Antioxidantien nicht erforderlich.

Wirkstoffzubereitungen der erfindungsgemäßen Zusammensetzung sind sicher, nicht toxisch, gut verträglich und zeigen eine Hautschutzwirkung, die auf Tocopherol bzw. Tocopherol-Derivate zurückzuführen ist. Verwießen sei auf die im großen Umfang verwendeten kosmetischen Zubereitungen, die exogene Hautschäden, beispielsweise durch UV-Bestrahlung verhüten sollen. Die erfindungsgemäßen Zubereitungen sind im Vergleich mit bekannten topischen Wirkstoffzubereitungen überlegen, da Adsorptionspromotoren oder unter Umständen nicht ungefährliche Salze vermieden werden, die zu Hautirritationen oder sogar zu Hautschäden führen können.

Aus den folgenden experimentellen Angaben ergibt sich, daß die Zugabe von Vitamin E (α-Tocopherol) bzw. eines seiner Derivate überraschenderweise die Permeation drastisch über den für ein Diethylammonium-Diclofenac-Gel geltenden Wert anhebt. So konnten Werte von bis zu etwa 4000 µg Wirkstoff/2,5 cm² ermittelt werden **(Tabelle 1).**

Nachstehend wird die Erfindung anhand von Beispielen und Figuren näher erläutert. Es zeigen:
- **Fig. 1**: ein Hautpermeationsprofil in vitro eines Diclofenac-Na-Gels des Stands der Technik;
- **Fig. 2**: ein Hautpermeationsprofil in vitro eines Diclofenac-Na-Gels gemäß der Erfindung; und
- **Fig. 3**: Plasmaspiegel als Funktion der Zeit bei einem Diclofenac-Na-Gel gemäß der Erfindung beziehungsweise des Stands der Technik.

### Vergleichsbeispiel 1 (Diethylammoniumdiclofenac)

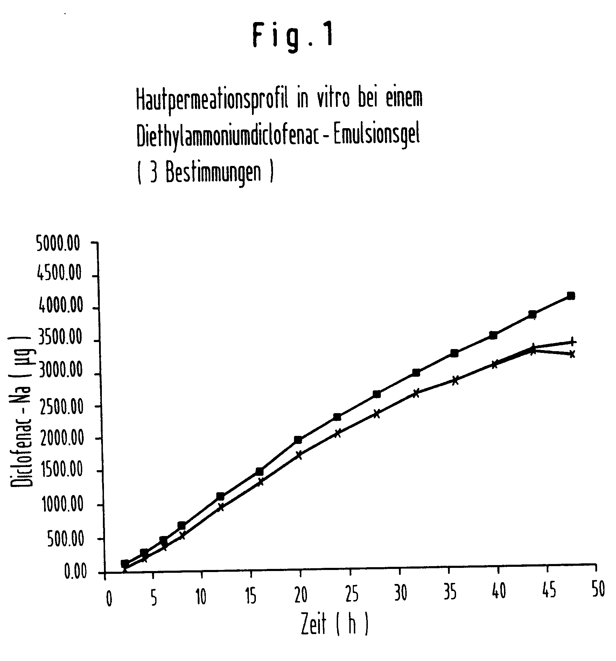

Es wurden Hautpermeationsuntersuchungen durchgeführt, wobei man herauspräparierte Haut nackter Mäuse in modifizierten Franzzellen verwendete. Die Permeationsfläche betrug 2,5 cm². Das Akzeptormedium war physiologische Kochsalzlösung bei 34 °C. Ein typisches Hautpermeationsprofil ist in **Figur 1** wiedergegeben. Ein im Handel erhältliches Emulgel (Voltaren) wurde als Bezug verwendet. Es enthält das Diethylammoniumsalz von Diclofenac. Alle permeierten Mengen wurden auf Basis von Diclofenac-natrium ausgedrückt. Aus diesem Versuch ließ sich ein ungefährer Fluß von J = 36 µg Wirkstoff/cm²/h berechnen. Das Diethylammoniumdiclofenac-Gel zeigte eine Permeation von etwa 2000 µg Wirkstoff/2,5 cm²/24 h.

### Vergleichsbeispiel 2 (Diclofenac-natrium)

Es wurde die Hautpermeation gesättigter wässeriger Lösungen und 1-proz. Lösungen für ein Gemisch von Isopropanol und Propylenglykol gemessen. Die Ergebnisse sind der folgenden **Tabelle 1** zu entnehmen. So betrug die Wirkstoffmenge, die *in vitro* durch die Haut in 24 Stunden bei einer gesättigten und wässerigen Lösung permeierte, 984 µg Wirkstoff/2,5 cm². Diese Lösung besaß maximale thermodynamische Aktivität. Eine 1-proz. wässerige Lösung mit einem Gehalt an Isopropylalkohol und Propylenglykol zeigte eine viel geringere Permeation von 135 µg Wirkstoff/2,5 cm². Die Zugabe von 2 % gereinigtem natürlichen Sojabohnenlecithin (circa 80 % Phosphatidylcholin, 2 % Lysophosphatidylcholin, 4 % Phosphatidsäure und 1 % Monophosphatidylinositol) erhöhte die Permeationsrate um einen Faktor von etwa 5 auf 750 µg Wirkstoff/2,5 cm², was etwa dem Wert einer gesättigten Lösung ohne Adsorptionspromotor entsprach. Phopholipide ohne Zugabe der Alkohole führten zu einem niedrigeren Wert von 230 µg Wirkstoff/2,5 cm².

**Tabelle 1**

| In 24 h diffundierte Mengen von Diclofenac-natrium (µg/2,5 cm²) in verschiedenen Lösungsmitteln | | | | |
|---|---|---|---|---|
| gesättigte wässerige Lösung | 1-proz. wässerige Lösung | 1 % in IPA/PG Wasser | 1 % in Wasser mit 2 % PL | 1 % in IPA/PG Wasser + 2 % PL |
| 984 | 398 | 150 | 230 | 750 |
| IPA: Isopropylalkohol PG : Propylenglykol PL : Phospholipid (wie vorstehend angegeben) | | | | |

Da die Löslichkeit in Wasser etwa 1,9 % beträgt, stimmen die Werte für die Hautpermeation für Lösungen in Wasser mit den Konzentrationsunterschieden überein. Die 1:1-Mischung von Isopropanol (IPA) und Propylenglykol (PG) mit 6 % Wasser scheint die Wirkstoffpermeation herabzusetzen. Auch die Verwendung des Phospholipids allein ohne Zusatz von Alkoholen reicht nicht aus, um die Permeation einer gesättigten Lösung in Wasser zu erreichen.

### Beispiel 1

Die Zugabe von α-Tocopherol oder α-Tocopherolacetat zur Lösung von Diclofenac-natrium als Wirkstoff, PL und Alkoholen führen zu einer unerwartet großen Hautpermeation (**Tabelle 2**). Die Wirkstofflösungen wurden mit Polyacrylsäure und Triethanolamin gelliert, um eine leicht applizierbare Zubereitung zu bilden. Die Hautpermeation wird durch diese Modifikation nur leicht beeinflußt (**Tabelle 2**).

**Tabelle 2**

| In 24 h diffundierte Mengen von Diclofenac-natrium (µg/2,5 cm²) nach Zugabe von PL, α-Tocopherol oder α-Tocopherolacetat und IPA. | |
|---|---|
| 1 % Diclofenac-Na-Lösung | 1 % Diclofenac-Na-Gel |
| 3711 | 3350 |

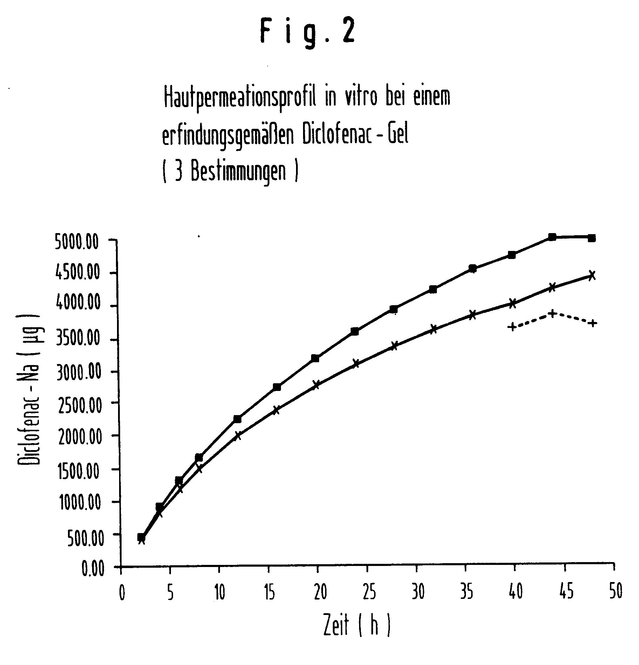

Beide Zubereitungen enthielten identische Mengen an PL und IPA. Die Lösung enthielt 5 % d,1-α-Tocopherolacetat und das Gel 1 % d-α-Tocopherol. Das Gel enthielt zusätzlich 1,5 % Polyacrylsäure und eine ausreichende Menge an Triethanolamin zum Gelieren des Polymeren. Um die Spreitung des Gels zu erhöhen, wurde eine Mischung aus synthetischen Ölkomponenten in das Gel eingearbeitet. Die Daten der Hautpermeation *in vitro* einer vollständigen Diclofenac-Na-Gel-Zubereitung sind **Figur 2** zu entnehmen.

Der sich aus diesen Daten errechnende Fluß beträgt J = 56 µg Wirkstoff/cm²/h. Er ist 1,55-mal höher als der einer Standardzubereitung.

### Anwendungsbeispiel 1 (Diclofenac-Na-Lösung)

Die folgende Lösung kann als topischer antirheumatischer Spray verwendet werden:

| | |
|---|---|
| Diclofenac-Na | 1 % |
| Phospholipon 80 | 2 % |
| d,l-α-Tocopherolacetat | 5 % |
| Isopropanol | 20 % |
| Wasser | 72 % |

### Anwendungsbeispiel 2 (Heparin-Na-Gel)

Das folgende Gel kann als topische antithrombotische Zubereitung verwendet werden:

| | |
|---|---|
| Heparin-Na | 0,5 % |
| Phospholipon 80 | 2 % |
| d-α-Tocopherol | 1 % |
| Polyacrylsäure | 1,5 % |
| Triethanolamin | 2,5 % |
| Isopropanol | 20 % |
| Wasser | 72,5 % |

### Anwendungsbeispiel 3 (Diclofenac-Na-Gel)

Das folgende Gel läßt sich sehr gut auf der Haut verteilen. Man kann es auf entzündeten Bereichen des Körpers auftragen.

| | |
|---|---|
| Diclofenac-Na | 1 % |
| Phospholipon 80 | 2 % |
| d-α-Tocopherol | 1 % |
| Polyacrylsäure | 1,5 % |
| Triethanolamin | 2,5 % |
| Decyloleat | 5 % |
| Isopropanol | 20 % |
| Wasser | 67 % |

### Anwendungsbeispiel 4 und Vergleichsanwendungsbeispiel 1 (Diclofenac-Na-Gele)

Es wurde Anwendungsbeispiel 3 wiederholt (Anwendungsbeispiel 4). Von dem 1-proz. Diclofenac-Na-Gel wurden jeweils 7,5 g auf die Rücken von 12 Probanden aufgetragen und nach 6-stündiger Applikationsdauer abgewaschen.

Zum Vergleich wurden jeweils 7,5 g eines handelsüblichen 1-proz. Diclofenac-Na-Gels (Voltaren) auf die Rücken von 12 Probanden aufgetragen, wobei man die Gele wiederum nach 6-stündiger Applikationsdauer abwusch.

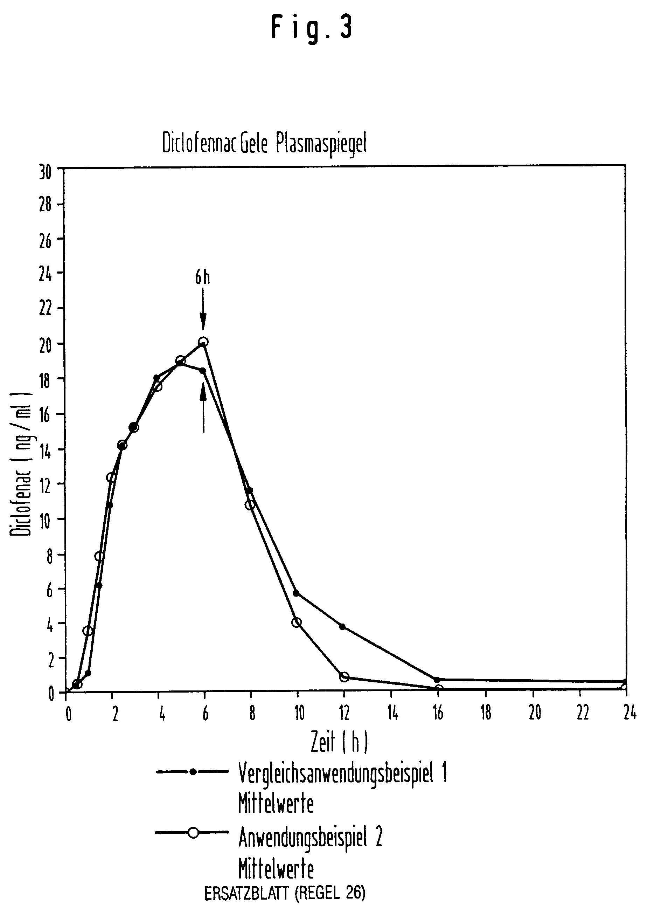

Die Mittelwerte der Plasmaspiegel sind in **Fig. 3** graphisch dargestellt. **Fig. 3** kann man entnehmen, daß beim Anwendungsbeispiel 4 bei Abbruch der Applikationen der Plasmaspiegel noch anstieg. Demgegenüber fiel beim Vergleichsanwendungsbeispiel 1 der Plasmaspiegel schon vor dem Abbruch der Applikationen bei etwa 5-stündiger Applikationsdauer ab. Dieser Abfall dürfte darauf zurückzuführen sein, daß das Vergleichsgel keinen Gehalt an Phospholipid und/oder α-Tocopherol aufweist.

## Patentansprüche

1. Wirkstoffzubereitung mit einem Gehalt an Phospholipid, dadurch **gekennzeichnet**, daß es sich um eine transdermale Wirkstoffzubereitung handelt, mit einem Gehalt an
- Wasser,
- einem aliphatischen C₁₋₆-Alkohol im Bereich von 1 bis 50 % (bezogen auf das Gesamtgewicht der Zubereitung), wobei 7,2 % und weniger ausgenommen sind,
- alpha-Tocopherol oder alpha-Tocopherol-Derivat und
- einem Hormon, Diclofenac-Na oder Heparin-Na als Wirkstoff.

2. Wirkstoffzubereitung nach Anspruch 1, **gekennzeichnet** durch einen Gehalt an α-Tocopherol oder α-Tocopherol-Derivat im Bereich von 0,1 bis 10 % und insbesondere 0,5 bis 7 %, bezogen auf das Gesamtgewicht der Zubereitung.

3. Wirkstoffzubereitung nach einem der vorhergehenden Ansprüche, **gekennzeichnet** durch einen aliphatischen C₂₋₄-Alkohol als aliphatischen C₁₋₆-Alkohol, gegebenenfalls neben einem weiteren üblichen Alkohol.

4. Wirkstoffzubereitung nach einem der vorhergehenden Ansprüche, **gekennzeichnet** durch einen Gehalt an Phospholipid im Bereich von 0,5 bis 20 %, bezogen auf das Gesamtgewicht der Zubereitung.

5. Wirkstoffzubereitung nach einem der vorhergehenden Ansprüche, **gekennzeichnet** durch Liposome auf Basis des Phopholipids.

6. Wirkstoffzubereitung nach einem der vorhergehenden Ansprüche, dadurch **gekennzeichnet**, daß sie als Salbe, Gel oder Spray vorliegt.

## Claims

1. Drug preparation containing phospholipid, characterized in that it is a transdermal drug preparation containing
- water,
- an aliphatic C₁₋₆-alcohol in the range from 1 to 50% (based on the total weight of the preparation), 7.2% and less being excluded,
- alpha-tocopherol or alpha-tocopherol derivative and
- a hormone, diclofenac Na or heparin Na as the drug.

2. Drug preparation according to Claim 1, characterized in that it contains α-tocopherol or α-tocopherol derivative in the range from 0.1 to 10% and in particular 0.5 to 7%, based on the total weight of the preparation.

3. Drug preparation according to one of the preceding claims, characterized by an aliphatic C₂₋₄-alcohol as the aliphatic C₁₋₆-alcohol, optionally in addition to a further customary alcohol.

4. Drug preparation according to one of the preceding claims, characterized in that it contains phospholipid in the range from 0.5 to 20%, based on the total weight of the preparation.

5. Drug preparation according to one of the preceding claims, characterized by liposomes based on the phospholipid.

6. Drug preparation according to one of the preceding claims, characterized in that it, is present as an ointment, gel or spray.

## Revendications

1. Composition d'un principe actif contenant des phospholipides, caractérisée en ce qu'il s'agit d'une composition de principe actif destinée à une administration transdermique, contenant :
- de l'eau,
- de 1 à 50 % (rapporté au poids total de la composition) d'un alcool aliphatique en C₁₋₆, des teneurs inférieures ou égales à 7,2 % étant exclues,
- de l'α-tocophérol ou un dérivé d'α-tocophérol, et
- une hormone, du diclofénac de sodium ou de l'héparine de sodium comme principe actif.

2. Composition d'un principe actif selon la revendication 1, caractérisée par le fait que la teneur en α-tocophérol ou en dérivé d'α-tocophérol est comprise entre 0,1 et 10 % en poids, en particulier entre 0,5 et 7 % en poids, rapporté au poids total de la composition.

3. Composition d'un principe actif selon une des revendications précédentes, caractérisée par le fait que l'alcool aliphatique en C₁₋₆ est un alcool aliphatique en C₂₋₄ et est éventuellement accompagné d'un autre alcool usuel.

4. Composition d'un principe actif selon une des revendications précédentes, caractérisée par le fait que la teneur en phospholipides est comprise entre 0,5 et 20 % en poids rapporté au poids total de la composition.

5. Composition d'un principe actif selon une des revendications précédentes, caractérisée par la présence de liposomes formés à partir des phospholipides.

6. Composition d'un principe actif selon une des revendications précédentes, caractérisée par le fait qu'elle se présente sous forme de pommade, de gel ou de spray.
